# EUROPEAN PATENT APPLICATION

(11) **EP 4 371 552 A1**
(43) Date of publication of application: **22.05.2024**
(21) Application number: 22842523.7
(22) Date of filing: 15.07.2022
(51) Int. Cl.: A61K 8/63, A61K 8/73, A61Q 19/06, A61K 9/00, A61K 31/575, A61K 31/728, A61P 3/04

(54) **COSMETIC COMPOSITION COMPRISING MOLECULAR AGGREGATES OF BILE ACIDS OR BILE ACID SALTS**

(30) Priority: 16.07.2021 KR 20210093764
(71) Applicant: Scai Therapeutics Co., Ltd., Seoul 06524 (KR)
(72) Inventor: KIM, Kyoung Hee, Daejeon 35246 (KR); KIM, Chul Hwan, Daejeon 35246 (KR); JANG, Ji Sung, Daejeon 34011 (KR)
(74) Representative: dompatent von Kreisler Selting Werner - Partnerschaft von Patent- und Rechtsanwälten mbB
(86) International application number: PCT/KR2022/010405
(87) International publication number: WO 2023/287261

(57) **Abstract**

The present invention relates to a cosmetic composition comprising a molecular association of bile acid and bile salt.

## Description

### [Technical Field]

The present invention relates to a cosmetic composition comprising a molecular association of bile acid or bile salt, and more specifically, to a cosmetic composition comprising a molecular association of bile acid or bile salt prepared by applying shear stress to a bile acid or bile salt that can be applied directly to the skin instead of a surgical method of directly injecting and delivering drugs using a needle or syringe, and has a superior locally reducing fat effect than existing products.

### [Background Art]

Bile acid or bile salt emulsifies fat and promote the action of lipase, a digestive enzyme, to dissolve fatty acids. Since it is also present in the digestive system of the human body, it plays a role in helping digestion and absorption by breaking down the ingested fat. Using this mechanism, Allergan, a global company, developed a fat removal injection using deoxycholic acid, a type of bile salt, and has received approval from the Food and Drug Administration of each country for its Belkyra^{™} (in Canada) products.

However, when injected with Belkyra^{™}, deoxycholic acid destroys fat cells so that fat cannot be stored any more, but other cells may also die in the process. Therefore, there was a hassle of having to be injected by a medical professional with anatomical knowledge. In addition, since it is an injectable formulation, it is accompanied by side effects such as pain, swelling, bruising, redness, and numbness of the face. If the side effects were severe, side effects such as facial muscle weakness, awkward facial expressions and facial movements, difficulty chewing with the jaw, and jaw nerve damage occurred.

Existing fat-reducing preparations using deoxycholic acid use synthetic deoxycholic acid, so the burden on manufacturers for product production will be significant. In addition, since it was developed as an injection formulation and administered to patients, the hassle and side effects of the administration method were considerable.

### (Patent Document 1) EP 2422789 B1

### [Disclosure]

### [Technical Problem]

An object of the present invention is to provide a molecular association having fat removal performance similar to that of a subcutaneous injection that applies skin permeable molecular association of bile acid or bile salt to the skin without using a surgical method of directly injecting and delivering the drug using a needle and syringe, and ensuring patient convenience without side effects of the injection.

Further, another object of the present invention is to provide a cosmetic composition comprising the molecular association of bile acid or bile salt and a method for manufacturing thereof.

### [Technical Solution]

The present invention provides a cosmetic composition comprising a molecular association in which bile acid molecule or bile salt molecules are physically bonded, wherein when the molecular association is formed in a composition containing water, the molecular association has an associated structure in the composition.

Further, one embodiment of the present invention may provide a cosmetic composition wherein the molecular association has the pH of exceeding 8.5 and less than 10.

Further, one embodiment of the present invention may provide a cosmetic composition wherein the average particle diameter of the molecular association is 1.0 to 10 nm or less.

Further, one embodiment of the present invention may provide a cosmetic composition wherein the molecular association is amorphous.

Further, one embodiment of the present invention may provide a cosmetic composition having a concentration change rate of exceeding 0 and less than 5% for 12 months under the condition of 4°C, 25°C and 45°C.

Further, one embodiment of the present invention may provide a cosmetic composition wherein the bile acid is any one selected from the group consisting of cholic acid, chenodeoxycholic acid, glycocholic acid, taurocholic acid, deoxycholic acid and lithocholic acid, and the bile salt is any one bile salt selected from the group consisting of cholic acid, chenodeoxycholic acid, glycocholic acid, taurocholic acid, deoxycholic acid and lithocholic acid.

Further, one embodiment of the present invention may provide a cosmetic composition for a skin-permeable locally reducing fat.

Further, one embodiment of the present invention may provide a cosmetic composition wherein the composition is applied and used as an external skin preparation, which is a non-surgical and no injection method.

Further, one embodiment of the present invention may provide a cosmetic composition wherein the composition includes the molecular association in an amount of 0.05 to 10% by weight.

Further, one embodiment of the present invention may provide a cosmetic composition wherein the composition further comprises at least one selected from the group consisting of hyaluronic acid, sodium hyaluronate, collagen, hyaluronic acid, glycerin, white tree ear mushroom extract, natto gum, 1,2-hexanediol and polysorbate 80.

Further, one embodiment of the present invention may provide a cosmetic composition wherein the composition is localized to a site selected from the group consisting of abdominal and cervial fat, upper thigh fat, forearm fat, visceral fat accumulation, fat after breast enlargement surgery, chest fat, fat spread around the arms, fat under the eyes, fat under the chin, hip fat, calf fat, back fat, thigh fat, ankle fat, cellulite and combinations thereof.

Further, one embodiment of the present invention may provide a codmetic composition wherein the composition is used in any one formulation selected from the group consisting of solution, suspension, emulsion, paste, gel, cream, ointment, unguent, spray, powder, thickened formulation and cataplasm.

Further, one embodiment of the present invention may provide a cosmetic composition wherein the composition has a bile acid concentration of 0.001 to 0.2 µg/ml measured in plasma 3 hours after application to the skin.

### [Advantageous Effects]

The present invention has the advantage of reducing the hassle of the administration method because it can be applied directly to the skin instead of a surgical method of directly injecting and delivering the drug using a needle and syringe. In addition, unlike existing product forms, it has the advantage of having an excellent effect on skin permeability, lipolysis and accumulation reduction despite not being an injection formulation.

In addition, since the skin gap is 80 nm, it is difficult for conventional pharmaceutical products to penetrate into the skin, but in the case of the present invention, since it has a small size of 1.0 to 10 nm, it has an effect of increasing skin permeability.

In addition, since nano-sized dispersed particles are easily exposed to aggregation or Ostwald ripening phenomenon, storage stability is low. On the other hand, the molecular association and composition of the present invention have a low change in content and number of particles at room temperature and accelerated conditions of 4°C and 45°C, so they have excellent stability.

In addition, the present invention has the advantage of not necessarily using a third material such as surfactants, micelles, cyclodextrins, lipids, albumin, water-soluble polymers, stabilizers/dispersants, and carriers such as nanoparticles and porous particles that has been used in addition to API and water in order to improve solubility in the existing technology.

### [Description of Drawings]

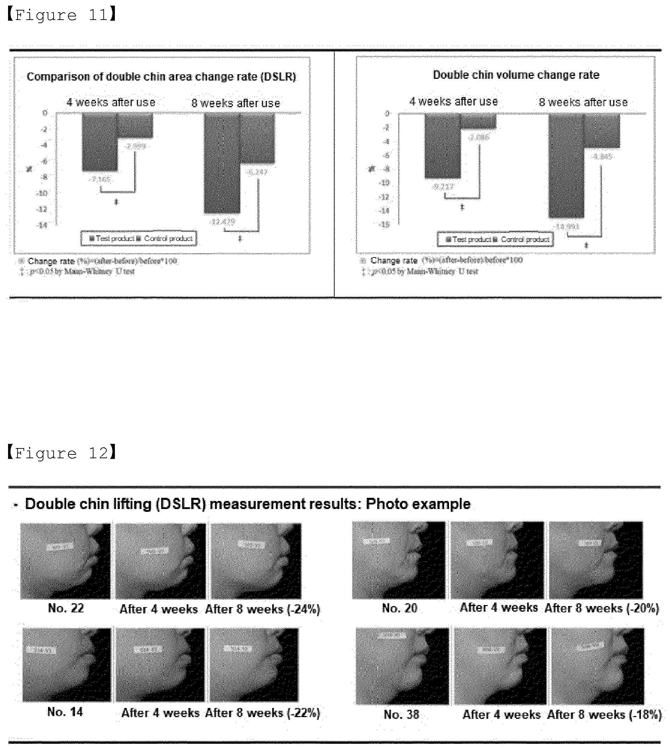

Fig. 1 is a TEM image of the molecular association of sodium deoxycholate according to one embodiment of the present invention.

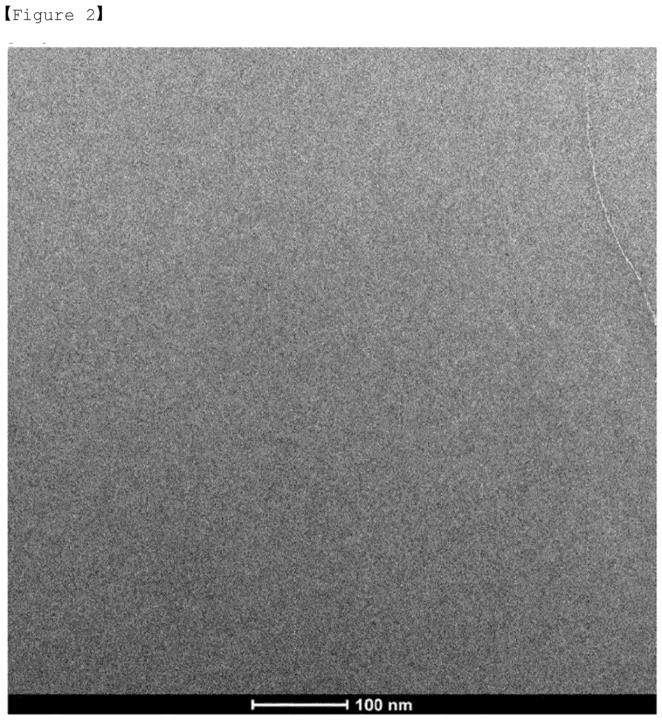

Fig. 2 is a TEM image of the sodium deoxycholate precursor according to one embodiment of the present invention.
Fig. 3 is a diagram of preadipocyte cytotoxicity of the molecular association of deoxycholic acid.
Fig. 4 shows the process of preadipocyte differentiation into adipocytes.
Fig. 5 is a diagram of adipocyte cytotoxicity of the molecular association of deoxycholic acid.
Fig. 6 is a diagram of measuring the amount of skin permeation by collecting the subcutaneous tissue after single injection of deoxycholic acid precursor into the subcutaneous tissue of a live mouse and continuous application of the same amount of the deoxycholic acid precursor and the deoxycholic acid molecular association of the present invention on the skin, respectively.
Fig. 7 is a diagram of comparing the amount of skin permeation using the amount of distribution in the skin tissue by collecting the subcutaneous tissue after single injection of deoxycholic acid precursor into the subcutaneous tissue of a live mouse and continuous application of the same amount of the deoxycholic acid precursor and the deoxycholic acid molecular association of the present invention on the skin, respectively.
Fig. 8 is a diagram comparing the amount of plasma distribution of the precursor of deoxycholic acid and molecular association over time after a single subcutaneous injection of deoxycholic acid precursor into the skin of a live mouse and continuous application of the same amount of the precursor and molecular association of deoxycholic acid to the skin.
Fig. 9 is the H&E staining result for evaluation of histological effects of the precursor of deoxycholic acid and molecular association on the subcutaneous tissue according to the application time after a single subcutaneous injection of deoxycholic acid precursor into the skin of a live mouse and continuous application of the same amount of the precursor and molecular association of deoxycholic acid to the skin.
Fig. 10 is the result of Masson's Trichrome staining for evaluation of histological effects of the precursor of deoxycholic acid and molecular association on the subcutaneous tissue according to the application time after a single subcutaneous injection of deoxycholic acid precursor into the skin of a live mouse and continuous application of the same amount of the precursor and molecular association of deoxycholic acid to the skin, and shows the increase in the area of the part stained in blue due to the destruction of adipocytes or the increase in collagen.
The left side of Fig. 11 is a diagram comparing the double chin area change rate when the molecular association of sodium deoxycholate according to one embodiment of the present invention is applied to the double chin for 4 weeks and 8 weeks, and the right side is a diagram showing the double chin volume change rate.
Fig. 12 is a diagram showing the changes observed for 8 weeks while applying the molecular association of sodium deoxycholate according to one embodiment of the present invention to the chin of a person.

### [Best Mode]

Poorly soluble drugs have extremely low saturated solubility in aqueous solution and extremely high interfacial tension/energy with water, so they are thermodynamically unstable, resulting in precipitation or phase separation. Therefore, in order to increase the drug content (i.e., solubility) in a pharmacologically meaningful way, a surface active agent capable of lowering the interface energy or a carrier capable of containing high content of drug was required.

In common, these existing solubility enhancement technologies either necessarily use the third material other than API and water or act as a key factor in improving solubility. For example, surfactants, micelles, cyclodextrins, lipids, albumin, water-soluble polymers, stabilizers/dispersants, nanoparticles, and porous particles fall under these third substances.

However, the inventors of the present invention prepare a molecular association utilizing polar interactions or hydrogen bonds so that the surface of the structure is hydrophobic without using the third material as above, thereby increasing the permeability to the phospholipid membrane, and adjust the particle diameter of the association to a level of 1.0 to 10 nm, thereby increasing the transmittance through skin gap having a size of about 80 nm. The inventors of the present invention have completed the present invention by confirming that the pharmacological substance is delivered to adipocytes in the skin through this to exhibit excellent effects on lipolysis and fat accumulation reduction.

It is explained in more detail below.

### Terms

In the present invention, the "bile acid" and "bile salt" means a steroid acid (and/or its carboxylic acid anion thereof), and salts thereof, which are found in the bile of animals (e.g., a human). Non-limiting examples thereof include any one bile acid selected from the group consisting of cholic acid, chenodeoxycholic acid, glycocholic acid, taurocholic acid, deoxycholic acid and lithocholic acid, or a bile salt thereof.

In the present invention, the "molecular association of bile acid or bile salt" refers to a molecular association prepared by applying shear stress to a solution containing bile acid so that bile acid molecule or bile salt molecules are physically bonded, and it means a structure in which bile acid molecules stick together.

In the present invention, the "bile acid molecule or bile salt molecules" refers to a precursor used to produce the molecular association of bile acid or bile salt according to the present invention, or the molecule itself of bile acid or bile salt as a precursor, and may also be referred to as "precursor". That is, the molecules of bile acid or bile salt according to the present invention refer to bile acid or bile salt to which shear stress is not applied.

In the present invention, the term "patient", "subject", "individual" and the like are used interchangeably herein, and refer to any animal, or cells thereof whether in vitro or in situ, amenable to the methods described herein. In certain non-limiting embodiments, the patient, subject or individual is a human.

In the present invention, the term "composition" refers to a mixture of at least one compound of the present invention with other chemical components, such as carriers, stabilizers, diluents, dispersing agents, suspending agents, thickening agents, and/or excipients.

In the present invention, the term "effective amount," "pharmaceutically effective amount" and "therapeutically effective amount" refer to a nontoxic but sufficient amount of an agent to provide the desired biological result. That result may be reduction and/or alleviation of the signs, symptoms, or causes of a disease, or any other desired alteration of a biological system. An appropriate therapeutic amount in any individual case may be determined by those of ordinary skill in the art using routine experimentation.

In the present invention, the term "efficacy" refers to the maximal effect (Eₘₐₓ) achieved within an assay.

In the present invention, the term "applying as an external skin application" refers to, for example, non-surgical and no injection method to apply a pharmaceutical composition to the outside of the skin to deliver the drug to the inside of the skin. In addition, it can be routinely determined by those skilled in the art in consideration of their knowledge and the present disclosure.

In the present invention, the term "local fat" refers to localization to a site selected from the group consisting of, for example, abdominal and cervial fat, upper thigh fat, forearm fat, visceral fat accumulation, fat after breast enlargement surgery, chest fat, fat spread around the arms, fat under the eyes, fat under the chin, hip fat, calf fat, back fat, thigh fat, ankle fat, cellulite and combinations thereof. In addition, it can be routinely determined by those skilled in the art in consideration of their knowledge and the present disclosure.

In the present invention, the term "external skin preparation" refers to, for example, a formulation that can be applied to the skin, and is used in any one formulation from the group consisting of gel, cream, ointment, unguent, spray, thickened formulation and cataplasm. In addition, it can be routinely determined by those skilled in the art in consideration of their knowledge and the present disclosure

### Molecular association of bile acid or bile salt

The present invention provides a molecular association in which bile acid molecule or bile salt molecules are physically bonded, wherein when the molecular association is formed in a composition containing water, the molecular association has an associated structure in the composition.

In the present invention, the average particle diameter of the molecular association may be 1.0 to 10 nm or less, preferably 1.5 nm or more, 2.0 nm or more, 7.0 nm or less, 5.0 nm or less, 3.0 nm or less. The average particle diameter of the molecular association can be measured through a diffraction experiment, preferably using a Zetasizer or Small Angle Neutron Scattering (SANS) . Also, images can be obtained using Transmission Electron Microscopy. When the average particle diameter of the molecular association exceeds 10 nm, there are problems in dispersibility, transparency and transmittance. In addition, the lower limit of the average particle diameter of the association is not particularly limited, but about 1.0 nm or more may be used.

As the molecular association according to the present invention is prepared by applying shear stress to bile acid molecule or bile salt molecules, it can have an amorphous shape even though it is manufactured in nano size, and accordingly, it is not only easy to adjust the size, but also has excellent skin permeability.

In addition, the molecular association according to the present invention may have the pH of exceeding 8.5 and less than 10. When the pH value satisfies the above range, the skin permeability of the molecular association is increased, and fat can be effectively decomposed after reaching adipocytes. In addition, the molecular association of the present invention may have a relatively high pH as it is used as a topical, unlike substances conventionally used as injections, and storage stability may be further improved as the pH is high. Specifically, the pH of the molecular association may be exceeding 8.6, exceeding 8.7, exceeding 8.8, exceeding 8.9, exceeding 9.0, exceeding 9.1, and less than 9.9, less than 9.8, less than 9.7, less than 9.6, less than 9.5, less than 9.4, less than 9.3.

Further, the molecular association according to the present invention has excellent storage stability. Unlike general nano-sized dispersed particles, which have low storage stability because they are easily exposed to aggregation or Ostwald ripening phenomenon, the molecular association and composition of the present invention have excellent stability due to a low concentration change rate of exceeding 0 and less than 5% for 12 months under the conditions of 4°C, 25°C and 45°C. The change rate means an average rate of change obtained by calculating an average value of change rates for each month up to the above period.

As described above, it can be seen that the stability of the molecular association according to the present invention is excellent.

### Method for manufacturing molecular association

The molecular association of bile acid or bile salt according to one embodiment of the present invention can be manufactured by applying shear stress to a solution containing the precursor of the molecular association, bile acid or bile salt.

The shear stress applied to the solution containing the precursor of the molecular association, bile acid or bile salt, may be either mechanical shear stress or ultrasonic application.

The mechanical shear stress may be applied by passing the solution through a silica-filled column or filter paper. Hereinafter, the mechanical shear stress will be described in detail.

According to one embodiment of the present invention, the mechanical shear stress may be applied by passing the solution containing the precursor of the molecular association, bile acid or bile salt, through a silica-filled column. When the solution containing bile acid or bile salt passes through a column filled with silica or the like, the precursor of the molecular association, bile acid or bile salt, is subjected to very high shear stress as it passes through a physically narrow area.

The silica may be spherical or prismatic, but its shape is not limited.

The average particle size of the silica may be 1.0 to 50 ***µ*m,** specifically 1.5 ***µ*m** or more, 2 ***µ*m** or more, and 40 ***µ*m** or less, 30***µ*m** or less, 20 ***µ*m** or less, 10 ***µ*m** or less, 5 ***µ*m** or less. When the size of the silica is less than 1.0 um or exceeding 50 um, even if the solution containing bile acid or bile salt passes through the column filled with silica, shear stress is not applied and thus molecular association may not change.

A negative pressure of 0.1 bar to 1.0 bar or 0.2 bar to 0.9 bar may be applied to the bottom of the silica-filled column. When the negative pressure applied to the bottom of the silica-filled column is less than 0.1 bar, the time required for the solution containing bile acid or bile salt to pass through the column increases, and thus the manufacturing time of the molecular association of bile acid or bile salt according to the present invention may be delayed. In addition, when the negative pressure applied to the bottom of the silica-filled column exceeds 1.0 bar, the time required for the solution containing bile acid or bile salt to pass through the column is reduced, thereby reducing the manufacturing time of the molecular association of bile acid or bile salt according to the present invention, but the manufacturing cost may increase because additional pump equipment is required.

According to another embodiment of the present invention, the mechanical shear stress may be applied by passing the solution containing bile acid or bile salt through one or more filter papers. When passing through the one or more filter papers, the precursor of the molecular association, bile acid or bile salt is subjected to very high shear stress by passing through a physically narrow area.

The filter paper may be one filter paper or a plurality of filter papers of two or more. When the filter paper is a plurality of filter papers of two or more, the filter papers may be stacked. When the filter paper is a plurality of filter papers of two or more, shear stress higher than that of one filter paper may be provided.

The size of pores of the filter paper may be 0.1 to 5.0 micron or 0.3 to 4.5 micron. When the pore size of the filter paper is less than 0.1 micron, the amount of the bile acid-containing solution passed through or filtered through the filter paper is too small, and thus the manufacturing speed of the molecular association of bile acid or bile salt according to the present invention may be reduced. When the pore size of the filter paper exceeds 5.0 micron, the bile acid-containing solution simply passes through the filter paper, and shear stress may not be effectively applied.

The shear stress may be applied using ultrasonic waves. Hereinafter, application of ultrasonic waves will be described in detail.

According to one embodiment of the present invention, the shear stress may be applied by applying ultrasonic waves to the solution containing bile acid or bile salt.

When the ultrasonic waves are applied to the solution containing bile acid or bile salt, pressure waves are generated, and the shear stress may be applied to bile acid or bile salt, which is a precursor of the molecular association, by the pressure wave.

The intensity of the applied ultrasonic waves may be 200 J/sec to 800 J/sec or 400 J/sec to 600 J/sec.

The energy applied per volume of the applied ultrasonic waves may be calculated as the intensity of the ultrasonic waves (J/sec) × the applied time (sec) / the measured volume (ml).

According to one embodiment of the present invention, Energy applied per volume of ultrasonic waves applied to the solution containing bile acid or bile salt may be 100 J/ml to 90 kJ/ml.

When the energy of the ultrasonic waves is less than 100 J/ml, it may be difficult to form the molecular association because sufficient shear stress is not applied to the solution containing bile acid or bile salt. In addition, when the energy of the ultrasonic waves exceeds 90 kJ/ml, it may be difficult to form the molecular association because excessive heat is applied to the solution containing bile acid or bile salt.

The ultrasonic waves may be applied for 10 seconds to 60 minutes at 10°C to 80°C. When the ultrasonic waves are applied at a temperature of less than 10°C, there is no change in the solution containing bile acid or bile salt, and when applied at a temperature exceeding 80°C, a phase change occurs in the solution containing bile acid or bile salt and the molecular association of bile acid or bile salt according to the present invention can be difficult. In addition, when the ultrasonic waves are applied for less than 10 seconds, there is no change in the solution containing bile acid or bile salt. When the ultrasound is applied for a time period of exceeding 60 minutes, the molecular association in the solution containing bile acid or bile salt is transformed, and the molecular association of bile acid or bile salt according to the present invention cannot be formed.

According to another embodiment of the present invention, as a method of applying the shear stress, a column filled with silica may be combined with an ultrasonic generator. The column filled with silica may be placed inside the ultrasonic generator, or the column filled with silica and the ultrasonic generator may be separated and continuously placed.

For example, after pouring the solution containing bile acid or bile salt into the column filled with silica, the column may be placed in the ultrasonic generator to apply ultrasonic waves. In addition, after ultrasonic waves are applied to the solution containing bile acid or bile salt, the solution may be passed through the column filled with silica.

### Cosmetic composition

The present invention provides a cosmetic composition comprising the molecular association. The cosmetic composition can be used for locally reducing fat.

In the present invention, the cosmetic composition may further comprise any one or more selected from the group consisting of hyaluronic acid, sodium hyaluronate, collagen, hyaluronic acid, glycerin, white tree ear mushroom extract, natto gum, 1,2-hexanediol and polysorbate 80 in addition to the molecular association.

Further, the cosmetic composition may further include components for use in a cream formulation, and these components are not particularly limited, and any deformable ones may be used. For example, it may further include any one or more selected from the group consisting of purified water, butylene glycol, propanediol, hydrogenated rice bran oil, caprylic/capric triglyceride, pentylene glycol, sodium deoxycholate, hyaluronic acid, dimethicone, hydrogenated Lecithin, hydroxyethyl acrylate/sodium acryloyldimethyltaurate copolymer, carbomer, sodium stearoyl glutamate, 1,2-hexanediol, hydrogenated rice bran oil, white trea ear mushroom extract, sodium Hyaluronate, natto Gum, Polysorbate 60, Xanthan Gum, fragrance, adenosine, sorbitan isostearate, Polysorbate 60, linalool, limonene and hexyl cinnamal.

In the present invention, the cosmetic composition may be included so that the molecular association is 0.05% by weight to 10.0% by weight, specifically 0.08% by weight or more, 0.1% by weight or more, 0.3% by weight or more, 0.5% by weight or more, 1.0% by weight or more, and 5% by weight or less, 3.0% by weight or less, 2.0% by weight or less, 1.0% by weight or less.

In the present invention, the cosmetic composition may have a concentration of 0.001 to 0.2 µg/ml, specifically 0.01 ***µ*g/mℓ** or more, 0.05 ***µ*g/mℓ** or more, 0.1 ***µ*g/mℓ** or more, and 0.18 ***µ*g/mℓ** or less, 0.15 ***µ*g/mℓ** or less, 0.12***µ*g/mℓ** or less of bile acid or bile salt measured in plasma 3 hours after application to the skin.

Hereinafter, the present invention will be described in more detail through examples of the present invention. It goes without saying that the present invention is not limited to these examples.

### [Example]

### Example 1. Molecular association of Sodium deoxycholate(NaDC)

18.0 g of sodium deoxycholate (NaDC, Sigma Aldrich #30970) was put in a 1L beaker and dissolved in 604 g of water using a magnetic bar. Silica gel 60 (Merck) 60.5g was put into a 400mL beaker containing 242g of 0.1M NaHCO₃ and mixed with a spatula. Wetted silica was packed while slowly pouring it into a vacuum filtration device (200 mbar pressure) equipped with a vacuum pump. The NaDC solution was added to the packed silica. Before the silica bed dried, 160 g of water was added in two portions of 80 g each. The outflow time was 1 hour in total, and after the outflow was completed, it was filtered using a 0.45um membrane filter. The filtered effluent was 808g. The concentration of this solution was 2.07% and the pH was 7.67. The recovery rate of sodium deoxycholate in this process was 93%.

### Example 2. Composition containing molecular association of Sodium deoxycholate

Since Example 1 above is in an aqueous solution, it tends to flow when applied to the skin, so there is a possibility that it is not sufficiently delivered into the skin. Therefore, in order to prepare the molecular association prepared in Example 1 as a formulation applicable to the skin, after adding 0.7% by weight of hyaluronic acid to 1.0% by weight of the molecular association, sodium hyaluronate, collagen, hyaluronic acid, glycerin, white tree ear mushroom extract, natto gum, 1,2-hexanediol and polysorbate 80 was additionally added as a whole component to prepare a cream type formulation.

### Comparative Example 1. Precursor of Sodium deoxycholate

Sodium deoxycholate itself, which did not pass through the silica in Example 1, was used.

### [Test Example]

### Test Example 1. Measurement of particle size of molecular association

The particle size of the colorless, odorless, and transparent liquid molecular association of sodium deoxycholate prepared in Example 1 was analyzed using the following Zetasizer and TEM.

### Zetasizer analysis

After filtering the sodium deoxycholate molecular association of Example 1 with a 0.2 um filter, the article size was measured 10 times under the conditions shown in Table 1 using a Zetasizer (Malvern, Nano ZSP). From the size distribution by volume, the average particle size was measured under the conditions shown in Table 1, and the results are shown in Table 2.

**[Table 1]**

| | | | |
|---|---|---|---|
| **Temperature** | 25 °C | **Duration Used** | 80 S |
| **Count rate** | 461.9 Kcps | **Measurement (set)** | 10 |
| **Cell Description** | Disposable sizing cuvette | **Attenuator** | 8 |

**[Table 2]**

| No | Storage condition | Particle size (nm) |
|---|---|---|
| Sample 1 | 4°C | 1.64 |
| | 25°C | 1.59 |
| | 45°C | 1.52 |
| Sample 2 | 4°C | 1.79 |
| | 25°C | 1.44 |
| | 45°C | 1.56 |

### TEM analysis

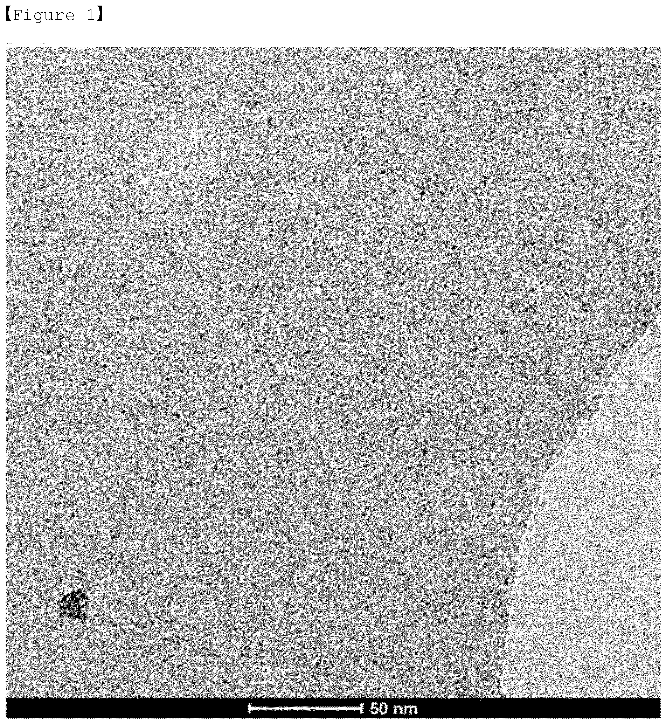

An EMS company's FCF300-Cu 50/pk (Formvar/Carbon 300Mesh, Copper) grid was placed on a filter paper, and about 20 µl of the sample was dropped on it with a micropipette. The grid was dried by waiting for 15 minutes or more without negative staining, and the particle size of the sodium deoxycholate molecular association of Example 1 was measured by TEM image. The results are shown in Figure 1

**[Table 3]**

| **Point resolution** | **Line resolution** | **HR STEM resolution** | **EDS resolution** |
|---|---|---|---|
| 0.205 nm | 0.102 nm | 0.16 nm | 136 eV |

As described above, it was confirmed that the molecular association had a particle size of 1.44 to 1.79 nm through Zetasizer and TEM analysis.

### Test Example 2. Stability test

### ① Materials

In order to confirm the storage stability of the molecular association of sodium deoxycholate to which the present invention was applied, the molecular association prepared in the same manner as in Example 1 was used as a stability test sample.

### ② Analysis method

### Content and particulate quantity analysis

The content and the number of particulates were analyzed by HPLC.

**[Table 4]**

| | |
|---|---|
| **Column** | RP C18 (215x4.6) |
| **Mobile phase** | Water: ACN : 85 % H₃PO₄ (50:50:0.1) |
| **Flow rate** | 1.0 mL/min |
| **Injection volume** | 10 µL |
| **Run time** | 17 min |
| **Wavelength** | 195 nm |
| **Sample Temp.** | 30°C |
| **Column Temp.** | 25°C |

### ③ Result

The molecular association prepared in Example 1 was taken as two samples, and changes were observed for 12 months at 4°C, room temperature and 45°C conditions, and the results are shown in Tables 5 and 6 below. As a result, in the case of the molecular association prepared in Example 1, no change over time was confirmed and it was found to be stable. Specifically, the change in concentration measured by HPLC was measured to be less than 5% for 12 months at 4°C, room temperature (25°C) and 45°C conditions. In addition, as a result of measuring the change in the number of insoluble particulates (number/ml), it was confirmed that all of them were maintained at a constant level for 12 months at 4°C, room temperature (25°C) and 45°C conditions.

**[Table 5]**

| No | Storage condition | Particulate size | Initial | | After 1 month | | After 2 months | |
|---|---|---|---|---|---|---|---|---|
| | | | HPLC content | Particulate number | HPLC content | Particulate number | HPLC content | Particulate number |
| Sample 1 | 4°C | <10*µ*m | 2.26% | <50/ml | 2.16% | <50/ml | 2.20% | <50/ml |
| | | <25*µ*m | | <5/ml | | <5/ml | | <5/ml |
| | | <50*µ*m | | <2/ml | | <2/ml | | <2/ml |
| | 25°C | <10*µ*m | 2.22% | <50/ml | 2.20% | <50/ml | 2.19% | <50/ml |
| | | <25*µ*m | | <5/ml | | <5/ml | | <5/ml |
| | | <50*µ*m | | <2/ml | | <2/ml | | <2/ml |
| | 45°C | <10*µ*m | 2.21% | <50/ml | 2.15% | <50/ml | 2.23% | <50/ml |
| | | <25*µ*m | | <5/ml | | <5/ml | | <5/ml |
| | | <50*µ*m | | <2/ml | | <2/ml | | <2/ml |
| Sample 2 | 4°C | <10*µ*m | 2.04% | <50/ml | 1.99% | <50/ml | 2.01% | <50/ml |
| | | <25*µ*m | | <5/ml | | <5/ml | | <5/ml |
| | | <50*µ*m | | <2/ml | | <2/ml | | <2/ml |
| | 25°C | <10*µ*m | 2.03% | <50/ml | 1.98% | <50/ml | 1.99% | <50/ml |
| | | <25*µ*m | | <5/ml | | <5/ml | | <5/ml |
| | | <50*µ*m | | <2/ml | | <2/ml | | <2/ml |
| | 45°C | <10*µ*m | 2.09% | <50/ml | 1.99% | <50/ml | 2.00% | <50/ml |
| | | <25*µ*m | | <5/ml | | <5/ml | | <5/ml |
| | | <50*µ*m | | <2/ml | | <2/ml | | <2/ml |

**[Table 6]**

| No | Storage condition | Particulate size | After 3 months | | After 6 months | | After 12 months | |
|---|---|---|---|---|---|---|---|---|
| | | | HPLC content | Particulate number | HPLC content | Particulate number | HPLC content | Particulate number |
| Sample 1 | 4°C | <10*µ*m | 2.16% | <50/ml | 2.21% | <50/ml | 2.18% | <50/ml |
| | | <25*µ*m | | <5/ml | | <5/ml | | <5/ml |
| | | <50*µ*m | | <2/ml | | <2/ml | | <2/ml |
| | 25°C | <10*µ*m | 2.16% | <50/ml | 2.15% | <50/ml | 2.19% | <50/ml |
| | | <25*µ*m | | <5/ml | | <5/ml | | <5/ml |
| | | <50*µ*m | | <2/ml | | <2/ml | | <2/ml |
| | 45°C | <10*µ*m | 2.15% | <50/ml | 2.18% | <50/ml | 2.17% | <50/ml |
| | | <25*µ*m | | <5/ml | | <5/ml | | <5/ml |
| | | <50*µ*m | | <2/ml | | <2/ml | | <2/ml |
| Sample 2 | 4°C | <10*µ*m | 2.08% | <50/ml | 2.05% | <50/ml | 2.06% | <50/ml |
| | | <25*µ*m | | <5/ml | | <5/ml | | <5/ml |
| | | <50*µ*m | | <2/ml | | <2/ml | | <2/ml |
| | 25°C | <10*µ*m | 2.09% | <50/ml | 2.08% | <50/ml | 2.05% | <50/ml |
| | | <25*µ*m | | <5/ml | | <5/ml | | <5/ml |
| | | <50*µ*m | | <2/ml | | <2/ml | | <2/ml |
| | 45°C | <10*µ*m | 2.10% | <50/ml | 2.09% | <50/ml | 2.02% | <50/ml |
| | | <25*µ*m | | <5/ml | | <5/ml | | <5/ml |
| | | <50*µ*m | | <2/ml | | <2/ml | | <2/ml |

### Test Example 3. Confirmation of cytotoxicity of preadipocytes and adipocytes

### ① Test material

To compare the cytotoxicity of preadipocytes and differentiated adipocyte, deoxycholic acid and deoxycholic acid molecular association (DCA2001JJ43, DCA2001JJ83-1) and deoxycholic acid salt molecular association (DCA2001JJ85-1) to which the present invention was applied were used.

### ② Preadipocyte cytotoxicity

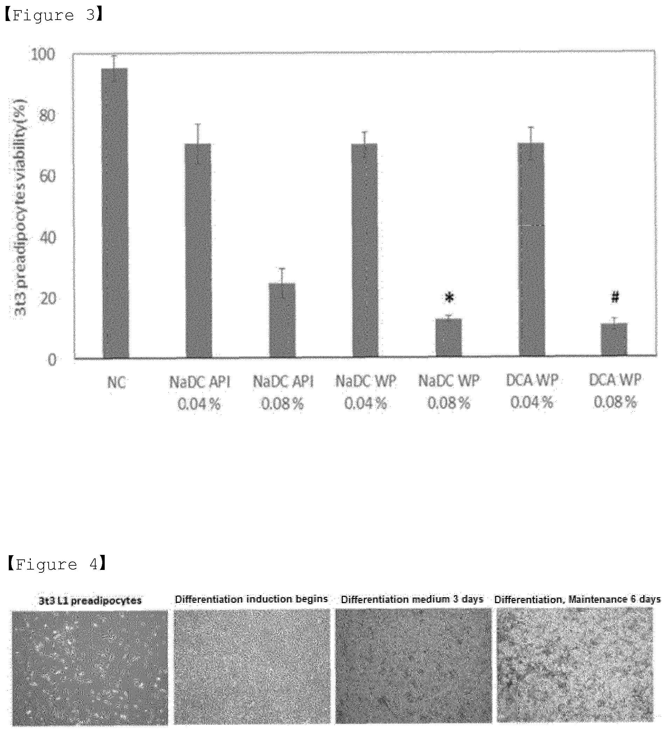

3T3-L1 preadipocytes were inoculated into a 96 well plate at 5×10³ per well, grown in medium (high glucose DMEM, 10% bovine calf serum, 1% penicillin/streptomycin) for 16 hours. The drug was prepared to contain 0.04% and 0.08%, respectively, and then treated for 4 hours. After adding 10 ul of Dojingo CK04-11 cell counting kit-8 to each well according to the manual, reacting for 2 hours, the absorbance at 450 nm was measured with a spectrophotometer to compare preadipocyte cytotoxicity. The results are shown in Fig. 3.

As a result, at 0.04%, the preadipocyte cytotoxicity of the precursor of deoxycholic acid salt and the molecular association of deoxycholic acid and the molecular association of deoxycholic acid salt were the same. However, when treated with 0.08%, compared to 24.6% of the precursor, the survival rate of preadipocytes to which the molecular association of deoxycholic acid salt was added decreased by 11.9% to 12.7% (*p=0.008), and in the group treated with the molecular association of deoxycholic acid, the survival rate decreased by 13.8% to 10.8% (#p=0.01). Therefore, the increase in preadipocyte cytotoxicity of the molecular association of deoxycholic acid to which the technology of the present invention was applied was confirmed.

### ③ Cytotoxicity of differentiated adipocyte

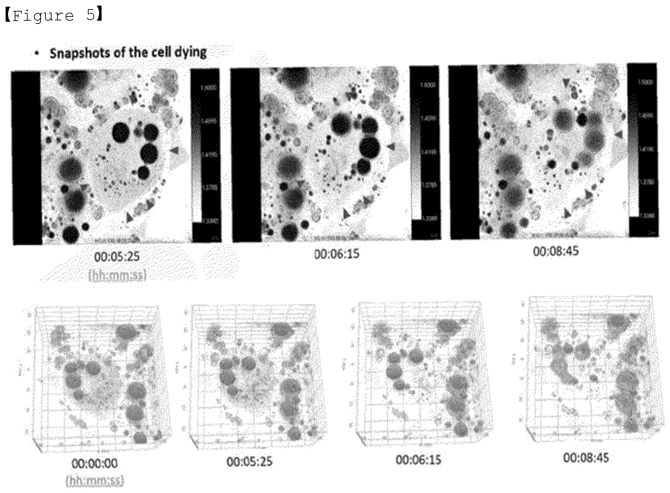

According to the Biovision 3T3 L1 differentiation kit manual, 3t3 L1 preadipocytes were grown to 100% confluence in a culture dish, and the culture medium was replaced with a differentiation maintenance medium. After 6 days, differentiated adipocyte with a large number of lipid droplets were induced (Fig. 4). Differentiated adipocytes were treated with 0.07% molecular association of deoxycholic acid(DCA2001JJ43). After acquiring 3D images at 2.5 frames per second using a Tomocube HT-2H microscope and applying the multi point acquisition function using imaging software TomoStudio, the changes in differentiated adipocytes were photographed at 25 second intervals for 40 minutes. As a result, the cell membrane and intracellular structure of the differentiated adipocyte, which were observed up to 5 minutes after imaging, rapidly faded after 5 minutes, and after 8 minutes, the RI value around the lipid droplet became almost the same as that of the external medium. From this, differentiated adipocyte cytotoxicity was confirmed (Fig. 5).

### Test Example 4. Preclinical trial

### ① SD rat skin penetration test

### Materials

An experiment was conducted to confirm the skin permeability and subcutaneous adipose tissue removal ability of the molecular association of deoxycholic acid (DCA2002JJ84 prepared by the method of Example 1) in SD rats.

### Test Method

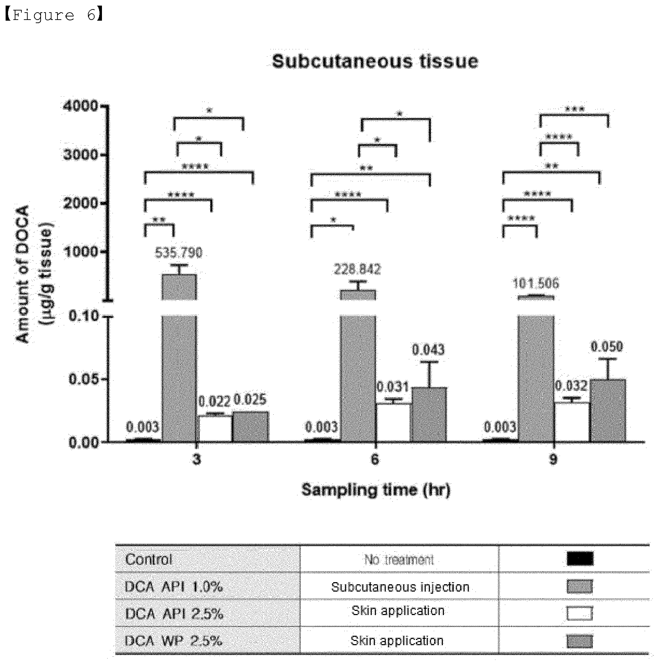

The interscapular region (back of the neck near the ear) of 10-week-old SD rats (280-350 g) with sufficient subcutaneous adipose tissue was shaved and anesthetized. A donor cell was attached to the shaved area with tape. 2.5% deoxycholic acid as a precursor and 500 ul of the deoxycholic acid molecular association of the present invention were added to the donor cells at 2.5%, and then applied to the skin continuously for 3, 6, and 9 hours under anesthesia. The distribution amount of the deoxycholic acid in subcutaneous tissue, skin, and plasma was checked to confirm skin permeability, and the cytotoxicity was confirmed in the subcutaneous fat cell layer through tissue staining, and the results are shown at the bottom of FIG. 6. A subcutaneous injection group of deoxycholic acid was used as a positive control group (1% DCA, 500 µl subcutaneous injection).

### Results of DCA distribution measurement in subcutaneous tissue

When the deoxycholic acid precursor and deoxycholic acid molecular association were continuously applied to the skin of SD rats, In both groups, an increase in skin permeability over time was confirmed by quantification of deoxycholic acid in the subcutaneous tissue. In the subcutaneous injection group, the amount of DCA gradually decreased in the subcutaneous tissue after subcutaneous injection, whereas in the skin application group, it increased over time, and the increase in transmittance of the deoxycholic acid molecular association was slightly higher than that of the deoxycholic acid precursor (Fig. 6).

### Result of measuring the amount of DCA distribution in the skin

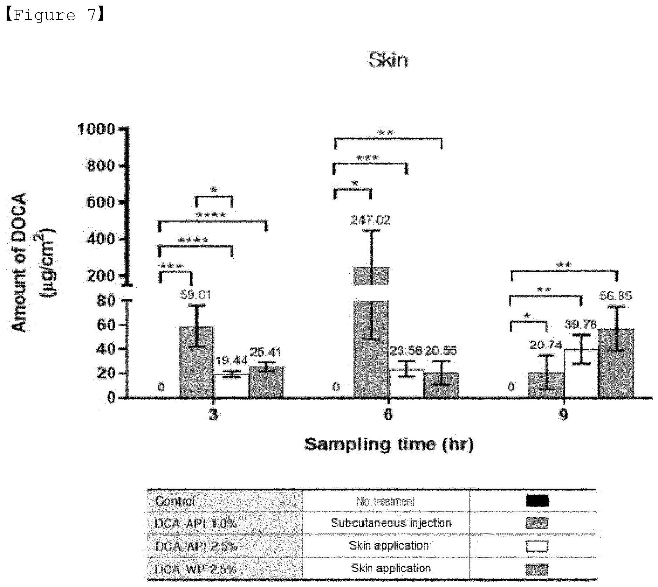

When the deoxycholic acid precursor and deoxycholic acid molecular association were continuously applied to the skin of SD rats, an increase in the deoxycholic acid distributed over the skin was confirmed in both groups over time. On the other hand, the subcutaneous injection group had the highest distribution of deoxycholic acid in the skin tissue after 6 hours, but showed a lower distribution than the skin application group at 9 hours (Fig. 7).

### Results of DCA distribution measurement in plasma

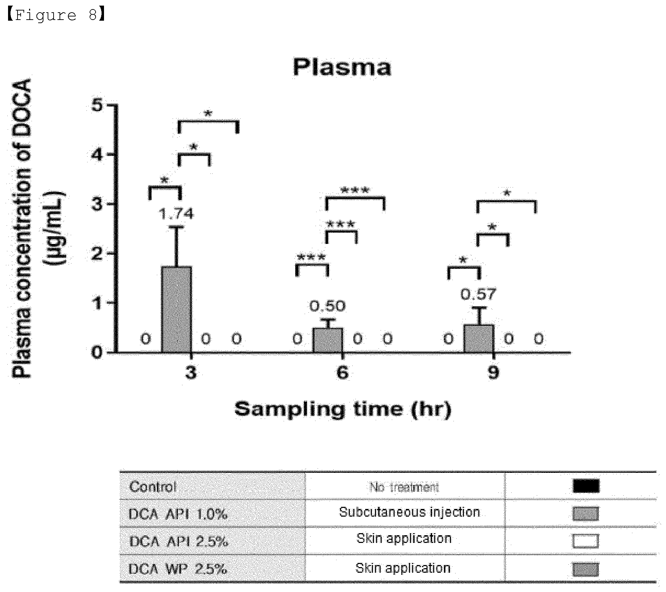

Fig. 8 shows the concentration of DCA in rat plasma. DCA was detected in plasma only in the deoxycholic acid subcutaneous injection group, but not in the skin application group. From this, it was confirmed that the deoxycholic acid molecular association has a subcutaneous fat removal effect by skin application, but does not transfer to plasma, so there is no safety problem.

### Results of histological analysis

500 µL of 1% deoxycholic acid was subcutaneously injected, and 500 µL of 2.5% deoxycholic acid and 500 µL of 2.5% deoxycholic acid molecular association were applied under the skin where the franz cells were attached, respectively, and applied continuously for 3, 6, and 9 hours. Then, tissues (skin, subcutaneous tissue) of the interscapular region were collected, fixed, and subjected to H&E staining and Masson's trichome staining. Tissue changes were investigated through microscopic observation.

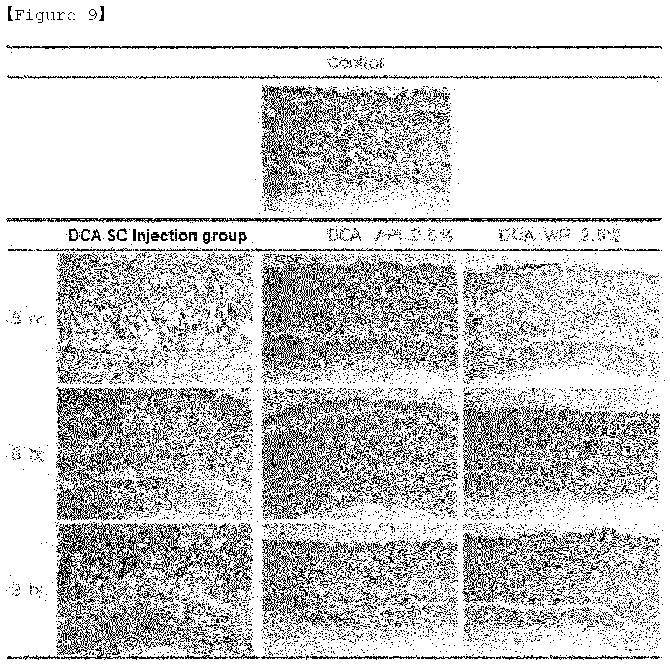

As a result of H&E staining, in the subcutaneous injection group of deoxycholic acid, severe tissue damage was observed after subcutaneous injection. In the skin application group, a decrease in dermis white adipose tissue was observed over time, and this was more clearly observed in the molecular association of deoxycholic acid application group (Fig. 9).

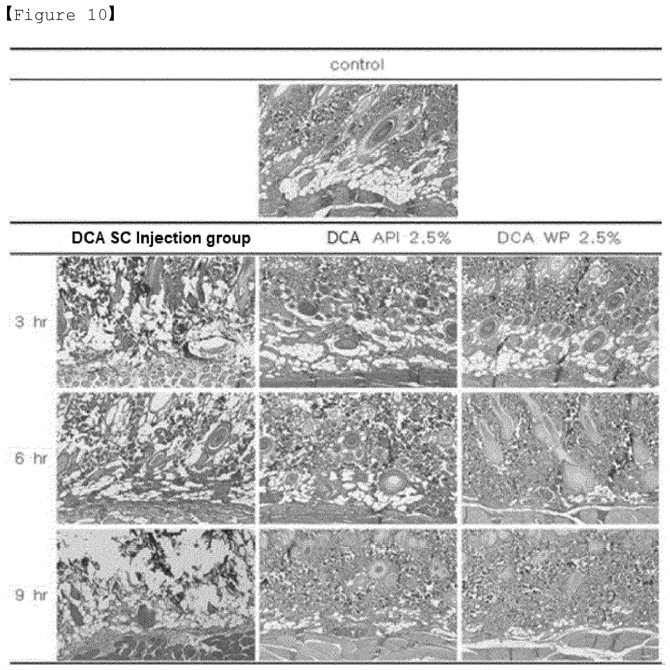

As a result of Masson's trichome staining to observe the increase or decrease of collagen in the skin and subcutaneous tissue, an increase in the area stained blue due to destruction of fat cells or increased collagen was observed in the skin application group (Fig. 10). In the subcutaneous injection group of deoxycholic acid, hematoma and edema occurred in the tissue, and the tissue was hardened during section creation due to severe tissue damage. Adequate tissue sections were not obtained in the 9 hr treatment group because the sections were split due to adipose tissue hardening.

### Conclusion

In the skin application group, the amount of deoxycholic acid distributed in the skin and subcutaneous tissue increased with time. The molecular association of deoxycholic acid showed a higher amount of deoxycholic acid distributed in the skin or subcutaneous tissue compared to the group treated with the precursor of deoxycholic acid. The concentration of deoxycholic acid in plasma was detected only in the subcutaneous injection group, and in the histological analysis, a decrease in adipose tissue was observed in the skin application group over time after administration. This phenomenon appeared more clearly in the molecular association of deoxycholic acid treatment group. As a result of the pharmacokinetic study, the amount of deoxycholic acid distributed in the subcutaneous tissue accumulated over time, and in histological analysis, the dermis white adipose tissue in the skin decreased. Therefore, it can be concluded that the molecular association of deoxycholic acid effectively penetrated the skin and contributed to the reduction of adipose tissue.

### Test Example 5. Double chin fat removal effect

The composition was applied to 42 women with an average age of 48.8 years old (21 in the test group and 21 in the control group) after washing their face in the evening once a day for 8 weeks from May 27 to July 22, 2020. The test group was treated with the cream formulation of Example 2 containing 1% of the molecular association of Example 1 of the present invention, and whole component were hyaluronic acid, sodium hyaluronate, collagen, hyaluronic acid, glycerin, white throat mushroom extract, natto gum, 1,2-hexanediol and polysorbate 80. As a control group, a cream containing sodium deoxycholate itself of Comparative Example 1 was used instead of the molecular association of Example 1. The effect of double chin lifting was compared between groups by measuring the area (pixel) in the image measured with a DSLR and the double chin volume value measured with the VECTRA XT device before, after 4 weeks, and after 8 weeks of use. In the test group using the cream of Example 1, a significant reduction in double chin area by 12% (p<0.05) and a significant reduction in double chin volume by 15% (p<0.05) were confirmed after 8 weeks of use. (Fig. 11 and Fig. 12)

There were no reports of special skin adverse reactions during the period of use of the test product by the subject, and according to the opinion of the dermatologist of the principal researcher, there were no abnormal findings in the test group and the use of 8 weeks helped improve the double chin lifting.

## Claims

1. A cosmetic composition comprising a molecular association in which bile acid molecule or bile salt molecules are physically bonded,
wherein when the molecular association is formed in a composition containing water,
the molecular association has an associated structure in the composition.

2. The cosmetic composition according to claim 1, wherein the average particle diameter of the molecular association is 1.0 to 10 nm or less.

3. The cosmetic composition according to claim 1, wherein the molecular association has the pH of exceeding 8.5 and less than 10.

4. The cosmetic composition according to claim 1, wherein the molecular association is amorphous.

5. The cosmetic composition according to claim 1, wherein the bile acid is any one selected from the group consisting of cholic acid, chenodeoxycholic acid, glycocholic acid, taurocholic acid, deoxycholic acid and lithocholic acid, and the bile salt is any one selected from the group consisting of cholic acid, chenodeoxycholic acid, glycocholic acid, taurocholic acid, deoxycholic acid and lithocholic acid.

6. The cosmetic composition according to claim 1, wherein the molecular association has a concentration change rate of exceeding 0 and less than 5% for 12 months under the condition of 4°C, 25 °C and 45 °C.

7. The cosmetic composition according to claim 1, wherein the cosmetic composition is for a skin-permeable locally reducing fat.

8. The cosmetic composition according to claim 1 or claim 7, wherein the composition is applied and used as an external skin preparation, which is a non-surgical and no injection method.

9. The cosmetic composition according to claim 1 or claim 7, wherein the composition includes the molecular association in an amount of 0.05 to 10% by weight.

10. The cosmetic composition according to claim 1 or claim 7, wherein the composition further comprises any one or more selected from the group consisting of hyaluronic acid, sodium hyaluronate, collagen, hyaluronic acid, glycerin, white tree ear mushroom extract, natto gum, 1,2-hexanediol and polysorbate 80.

11. The cosmetic composition according to claim 1 or claim 7, wherein the composition is localized to a site selected from the group consisting of abdominal and cervial fat, upper thigh fat, forearm fat, visceral fat accumulation, fat after breast enlargement surgery, chest fat, fat spread around the arms, fat under the eyes, fat under the chin, hip fat, calf fat, back fat, thigh fat, ankle fat, cellulite and combinations thereof.

12. The cosmetic composition according to claim 1 or claim 7, wherein the composition is used in any one formulation selected from the group consisting of solution, suspension, emulsion, paste, gel, cream, lotion, ointment, unguent, spray, powder, thickened formulation and cataplasm.

13. The cosmetic composition according to claim 1 or claim 7, wherein the composition has a bile acid concentration of 0.001 to 0.2 µg/ml measured in plasma 3 hours after application to the skin.
